# EUROPEAN PATENT APPLICATION

(11) **EP 1 985 259 A1**
(43) Date of publication of application: **29.10.2008**
(21) Application number: 07106697.1
(22) Date of filing: 23.04.2007
(51) Int. Cl.: A61F 2/24

(54) **Prosthetic heart valve holder**

(71) Applicant: SORIN BIOMEDICA CARDIO S.R.L., 13040 Saluggia (Vercelli) (IT)
(72) Inventor: Giaquinta, Giovanni, 10147, Torino (IT); Carlino, Felice Giuseppe, 10031, Borgomasino (Torino) (IT); Stacchino, Carla, 10132, Torino (IT)
(74) Representative: Bosotti, Luciano

(57) **Abstract**

A holder (10) for prosthetic heart valves including an annular base (18) and prosthetic valve leaflets extending between commissure regions oriented axially with respect to the base. The holder includes a grip element (12) from which a plurality of arms (14) extend for supporting the prosthetic heart valve in an intermediate position between the commissures. Protecting formations (22) extend radially with respect to the grip element to protect the commissure regions. The grip element includes a first rotational element (30) for coupling to a handle and at least one second coupling element (34) for coupling the grip element of the holder to a support element thereby impeding its rotation.

## Description

### TECHNICAL FIELD

The present invention relates to holders for implantable devices and was developed with particular attention paid to holders for prosthetic heart valves.

### BACKGROUND

Holders are used for supporting an implantable device, such as a prosthetic heart valve, before and during implantation. The holder may, for example, support the prosthesis (1) during production of the prosthesis, while the prosthesis is subjected to phases of immersion in treatment liquids (e.g., for the removal of undesired chemical residues from the prosthetic valve leaflets of biological tissue with procedures such as that described in EP-B-0 401 199), (2) after the valve has been inserted into its sterile package, where it is usually kept immersed in a storage liquid, and (3) during implantation, when the holder is coupled to a handle to enable the surgeon to implant the valve.

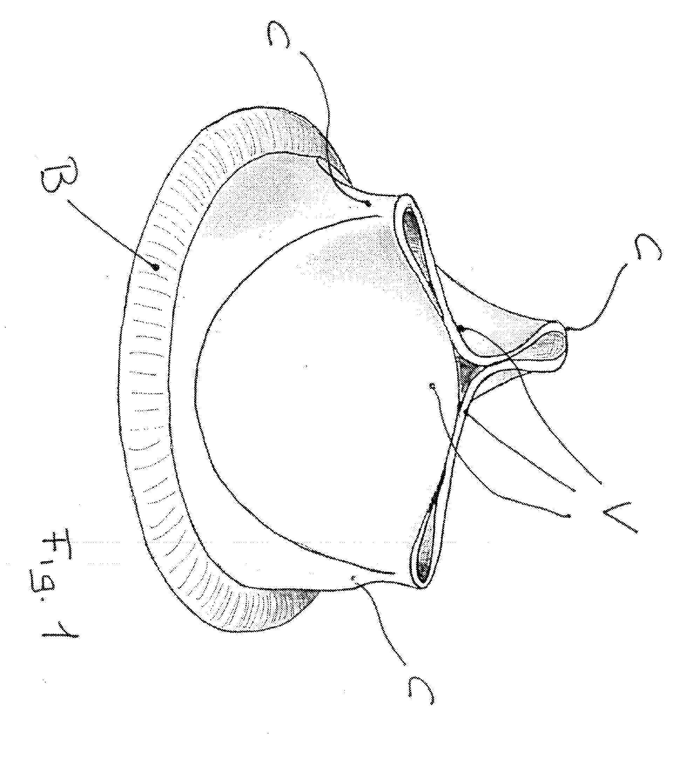

Figure 1 of the attached drawings shows an exemplary prosthetic tissue heart valve. One example of such a valve is disclosed in EP-A-0 155 245 and EP-B-0 515 324, both of which are hereby incorporated by reference. The valve, which may be either "stented" or "stentless," typically includes: an annular base region B, having an overall circular shape for suturing onto the valve annulus, and a series of prosthetic valve leaflets V that usually include a plurality of commissure regions C oriented in a generally axial direction with regard to the base region B of the valve. The proximal edges of the prosthetic valve leaflets are configured to mate when the prosthetic valve is the closed position, thus impeding blood flow through the valve. The mating leaflet surfaces thus define coapting edges of the leaflets in the closed position. Typically, in an effort to reproduce the structure of natural heart valves (e.g., aortic heart valves), the prosthetic valve has three commissures C between which three prosthetic valve leaflets V extend. The commissures C are thus generally spaced at 120° apart about the valve circumference.

Valve holder known in the art typically include a central grip element from which three arms (or struts) branch out and extend a sufficient distance to approximately envelope the profile of the valve. These arms are configured to attach to the valve at base region B. Often, the distal ends of the arms are connected together by a circular structure, which provides a support for the base region B of the valve. Typically, these arms extend downwardly adjacent the commissures C of the valve, so as to protect the commissures C during valve implantation.

These types of holders, which result in substantial contact between the holder and the prosthetic tissue valve, can have an undesirable impact on the tissue valve if such contact continues for a substantial length of time. Thus, there is a need in the art for a holder design that minimizes this contact.

### SUMMARY

The present invention, according to one embodiment, is a holder for a prosthetic heart valve, the valve including an annular base and a plurality of leaflets extending between commissure regions. The holder includes a grip element, a plurality of arms extending from the grip element and configured for supporting the prosthetic heart valve in an intermediate position between the commissure regions, and a plurality of commissure formations extending from the grip element. The formations are configured to extend radially from the grip element to cover an outward most point of the commissure regions.

The present invention, according to another embodiment, is a holder for a prosthetic heart valve, the valve including an annular base and a plurality of leaflets adapted to mate at coapting edges. The holder includes a grip element and a plurality of formations extending radially from the grip element, wherein the formations are configured to protect the coapting edges.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

Figure 1 is a schematic view of a prosthetic tissue heart valve, as is known in the prior art.

Figure 2 is a general perspective view of a holder, according to one embodiment of the present invention, coupled to a prosthetic valve of the type shown in FIG. 1 mounted onto it, and

Figures 3-5 illustrate different ways of using the holder described here.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims

### DETAILED DESCRIPTION

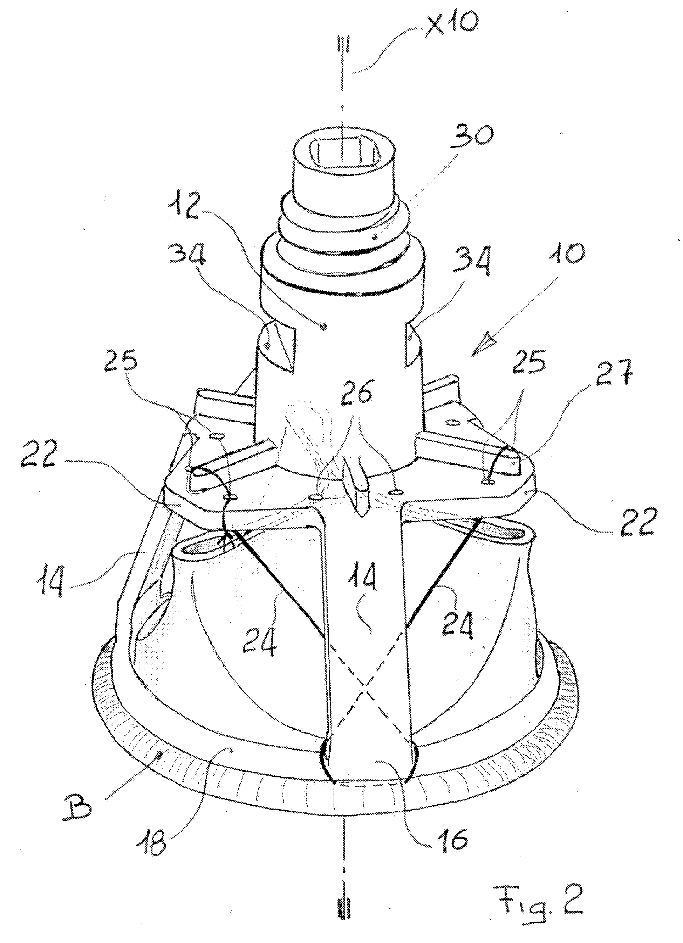

Figures 2-5 show a holder 10 configured for use with a prosthetic tissue heart valve of the type shown in figure 1. As shown in Figure 2, the holder 10 includes a grip or hub element 12, provided with coupling formations (further described below) and a plurality of arms 14 (e.g., three in number) that branch in a radial and distal direction from the grip element 12 to support the base region B of the prosthetic valve. As shown, the distal ends 16 of the arms 14 are linked together by a circular annular support formation 18, which is configured to support the base region B.

According to one embodiment, the support formation 18 includes an annular formation different from the continuous annular formation shown in figure 2. The support formation 18, for example, may include successive, distinct and separate stretches, of an overall annular structure, each stretch coupled to the distal end 16 of the respective arm 14, according to a general cross-bow-shaped configuration.

The holder 10 typically includes three arms 14, configured to extend between each of the three prosthetic valve commissures C. According to various embodiments, the holder includes more or less than three arms 14. As shown in figure 2, the holder 10 is suited to be coupled to the valve in such a manner that the arms 14 extend in an intermediate position between the commissures C. According to one embodiment, the arms 14 extend in an approximately central position with respect to the valve leaflets V, each of which presents a general eye-lid-like shape. The arms 14 are thus disposed so as to avoid contact with the commissures C. The valve V may be fastened onto the holder 10, according to any known technique, by employing suture threads 24 that extend from holes 25, 26 provided in the structure of the holder 10 and are passed around the valve in correspondence with the base region B.

In one embodiment, the holder 10 consists of a single piece of moulded material, of a type approved for use in the surgical field. In one embodiment, for reasons further described below, the material is a transparent or translucent material (e.g., polycarbonate). The terms "transparent" or "translucent" as used herein indicate a material that, due to its nature and/or morphology (thickness), does not impede the view of an element immediately below the material.

The valve 10 further includes canopy or commissure formations 22 that extend from the grip element 12 (e.g., from the root or proximal region of the arms 14). According to various embodiment, each of the canopy formations 22 projects in a radial direction with respect to the principal axis X10 of the holder 10 by an amount substantially sufficient to cover the radial dimension of the valve V. In other words, the distal end of each canopy formation 22 extends a sufficient distance to cover a respective commissure C.

According to some embodiments, the general V-shaped configuration of each canopy formation 22 is co-ordinated one to the next in such a manner that the set of the three canopy formations 22 forms, in correspondence with an ideal plane normal to the axis X10, a triangular structure. This triangular structure may have one or more blunt or cropped vertices. According to other embodiments, the formations 22 have alternative profiles. The formations 22, for example may have a generally finger-nail or petal shape configurations.

The canopy formations 22 protect the commissures C so as to impede, during implantation of the valve, the accidental and undesired extension of suture stitches in correspondence with the commissures C. The formations 22 protect the commissures C and serve to warn the surgeon against an adverse suture trajectory. The presence of the canopy formations 22, however, will not hinder the surgeon's view of the commissures C, if the canopy formations 22 are made of transparent or translucent material, such as for example polycarbonate. In this way, the commissures C are effectively protected against an undesired suturing action, but the commissures are nevertheless clearly visible to the surgeon. Preferably, the formations 22 are configured to cover or protect the entirety of the "coapting edges" of the prosthetic valve leaflets (as previously defined).

Preferably, according to various embodiments, the arms 14 are of sufficient length, and extend a sufficient distance in a downward direction, to allow for a space or gap between the formations 22 and the upper portions of the valve commissures C. This space minimizes or avoid contact between the formations 22, the coapting edges of the valve leaflets and the commissures C, which in turn minimizes or prevents exposure of the prosthetic tissue valve V to the material of the holder 10. This, in turn, avoids those undesired phenomena due to the pressure applied on the prosthetic tissue valve by the contact with the holder, as was described above.

During implantation, after suturing the valve (at least in a temporary manner) to the annulus, the surgeon may cut those stitches that fasten the valve onto the holder 10, such that when the surgeon separates the holder 10 from the valve, these fastening stitches will be removed with the holder 10 without running the risk of remaining in an undesired manner on the valve or at the implantation site.

According to some embodiments, the canopy formations 22 include, on their "proximal" face (i.e., the face directed away from the valve), ribs 27 which are in relief with regard to the general plane of the canopy formation 22. The ribs 27 are configured such that the fastening stitches applied using the openings 25 and/or 26 extend in a bridge-like fashion from the face of the canopy formations 22. This creates a space between the suture and the face, which facilitates removal by the surgeon.

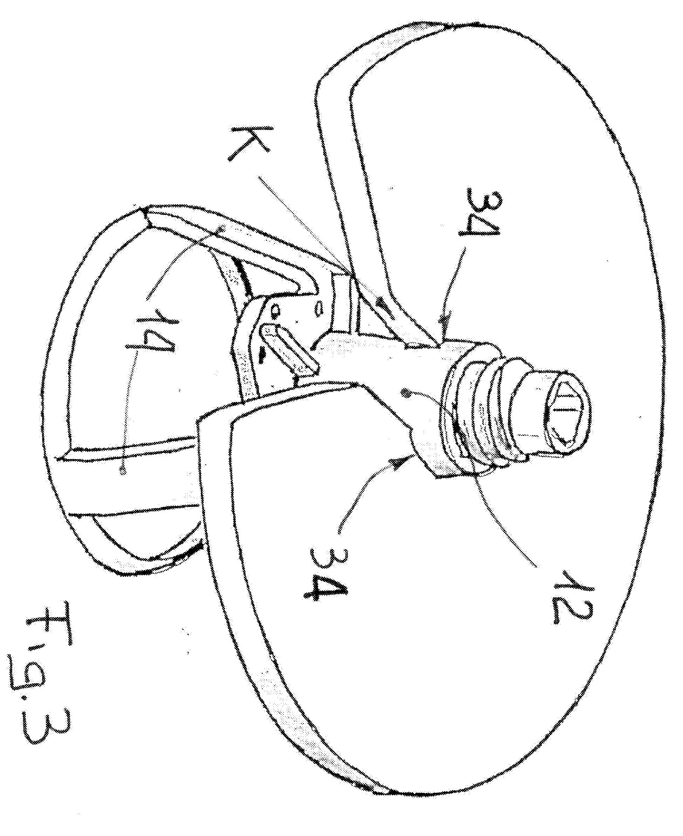

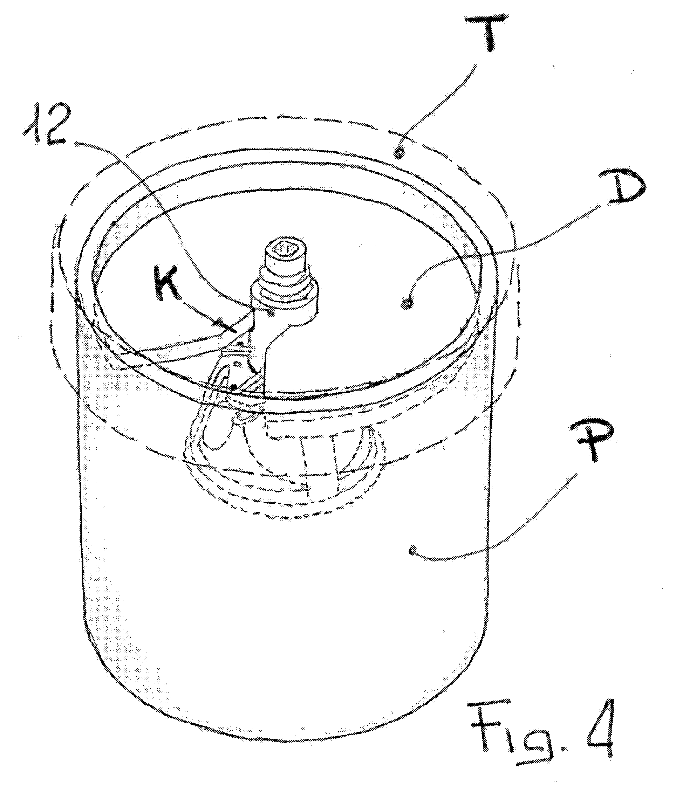

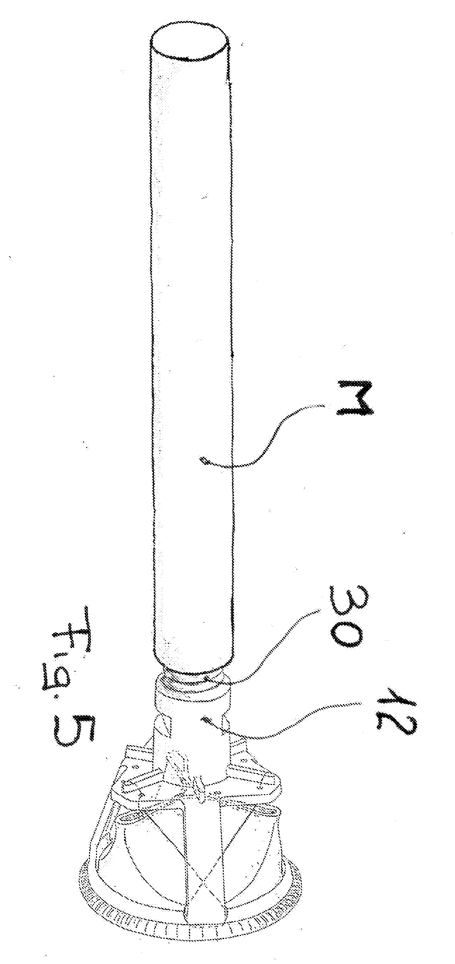

The sequence of figures 3, 4 and 5 shows, in schematic form, three successive operational conditions in which the holder 10 performs the function of supporting the valve. Figure 3 shows the function in which the holder 10 is supporting the valve mounted onto it in a treatment bath during the production phase of the valve. Figure 4 shows the holder 10 used to support the valve inside its sterile package, where the valve is once again usually immersed in a liquid, consisting of a storage bath. Lastly, figure 5 shows the holder 10 fixed to the distal end of a handle M, which is used by the surgeon to position and hold the valve on its insertion annulus during the implantation procedure.

These three different conditions of use must in general meet different requirements. In the condition shown in figure 3, it must be possible to transfer the holder 10 (and the valve mounted onto it) with relative ease, employing gripping devices, possibly robotic, thus without performing particularly complex movements. In the condition of use in figure 5 (implantation phase) it is imperative to ensure as solid and precise as possible a connection between the holder 10 (and the valve) and the handle M.

The condition shown in figure 4, on the contrary, represents an intermediate situation between the situation in figure 3 and the situation in figure 5. In particular, it is desirable that the holder 10, with the valve mounted onto it, can be inserted with relative ease into the sterile package, and that likewise it can be extracted with ease, in a precise and reliable way from the package, coupled to the handle M for subsequent use by the surgeon: all of this with a sequence of operations to extract it from the package and mount it onto the handle that are normally performed at the operating field by a surgical technician.

The solution described herein reconciles these different requirements, by fitting the grip element 12 with two coupling systems.

A first coupling system consists of a threaded rod 30 that projects at the opposite end of the grip element 12 with regard to the arms 14. The threaded rod 30 enables the holder 10 (and the valve that is mounted onto it) to be coupled to the handle M with a movement that is simple to perform, that is operating such that the handle M is screwed onto the threaded rod 30 of the holder 10. At the same time, this screw-thread coupling is very solid and precise. It is possible to operate in such a manner that this screw-thread coupling has an end-of-run limit position that is exactly predetermined from the angular standpoint, such that the valve will be in a precisely-determined angular position with respect to, for example, a landmark provided on the handle M.

The screw-thread coupling described might also be made in a complementary manner, that is by providing, at the end of the grip element 12 of the holder 10, a female screw-thread into which a threaded rod provided on the handle M could be screwed. The solution illustrated here (threaded rod on the holder 10 and not on the handle) appears however at the moment to be preferable since it avoids having to produce a recessed part (the female screw-thread) on a component, namely the holder 10, destined to be involved in the sterile packaging cycle of the valve.

Additionally, as shown in figure 3, the grip element 12 includes one or two notches 34 in a generally proximal position with respect to the screw-thread coupling element 30. The notch or notches 34 enable the grip element 12 of the holder 10 to be coupled with a corresponding support element through a coupling mechanism that in general may be defined as "through lateral translation with blocking of the rotation movement" of the grip element 12. In other words, the coupling mechanism comes about by effect of the lateral translation of the grip element 12, thus in the sense of a lateral translation with respect to the principal axis X10 of the holder 10, and at the same time impedes rotation of the grip element 12 (and of the holder 10 overall) around the axis X10.

The notch or notches 34 allow insertion of the grip element 12 into a groove-like reception element such as the element K shown in both figure 3 and figure 4. As shown in figure 4, the groove-like grip element K may simply comprise a notch in a discoid element D that rests on the rim of the sterile package P of the valve. This package generally presents in the form of a jar with a cup-like body and a screw-cap T (shown in dotted lines in figure 4).

A similar grip element may also be easily adopted for use in packaging and treatment of the valve, either in the form of a groove-like incision like that shown in figure 5, or again in the form of two jaws with a straight grip formation capable of penetrating into the groove or grooves 34. In the embodiment included a jaw, this coupling resists rotation of the grip element 12 about the axis X10, but does not require a lateral coupling movement of the type described above.

It follows that, without prejudice to the underlying principle of the invention, the details and embodiments may be varied, even significantly, with regard to what has been described and shown by way of non limiting example, without thereby departing from the scope of the invention, as is defined in the annexed claims. Likewise, various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

## Claims

1. A holder for a prosthetic heart valve, the valve including an annular base and a plurality of leaflets extending between commissure regions, the holder comprising a grip element, a plurality of arms extending from the grip element and configured for supporting the prosthetic heart valve in an intermediate position between the commissure regions, and a plurality of commissure formations extending from the grip element, the formations configured to extend radially from the grip element to cover an outward most point of the commissure regions.

2. The holder of claim 1, further comprising a support formation coupled to distal ends of the arms, the annular formation configured to support the base.

3. The holder of claim 2, wherein the support formation is substantially continuous.

4. The holder of claim 1, wherein the commissure formations are made of a transparent or a translucent material.

5. The holder of claim 1, wherein the commissure formations have a shape configuration selected from the group consisting of: a V-shape, a finger-nail-shape, and a petal-shape.

6. The holder of claim 4, wherein the plurality of commissure formations jointly form a substantially-triangular structure.

7. The holder of claim 1, further including openings to pass suture threads therethrough to fasten the valve onto the holder.

8. The holder of claim 7, wherein the openings are provided in correspondence with the arms.

9. The holder of claim 7, wherein the openings are provided in correspondence with the commissure formations.

10. The holder of claim 7, wherein the openings are provided both in correspondence with the arms and in correspondence with the commissure formations.

11. The holder of claim 1, wherein the commissure formations include projecting ridges on a proximal face.

12. The holder of claim 1, wherein the grip element includes:
at least one first rotational element for coupling the grip element to a handle, and
at least one second element for coupling the grip element to a support element configured to impede rotation of the grip element.

13. The holder of claim 12, wherein the at least one first coupling element is a screw-thread coupling element.

14. The holder of claim 13, wherein the screw-thread coupling element is a threaded rod.

15. The holder of claim 12, wherein the at least one second coupling element includes at least one lateral notch on the grip element.

16. The holder of claim 1, wherein the arms extend downwardly from the grip element a sufficient distance to effect a gap between the valve commissures and the commissure formations, upon attachment of the holder to the valve.

17. A holder for a prosthetic heart valve, the valve including an annular base and a plurality of leaflets adapted to mate at coapting edges, the holder comprising a grip element and a plurality of formations extending radially from the grip element, wherein the formations are configured to protect the coapting edges.

18. The holder of claim 17 wherein the formations are further configured to avoid contacting the coapting edges, while the prosthetic heart valve is secured to the holder.
